(19) Europäisches Patentamt
European Patent Office
Office européen des brevets

(11) **EP 4 142 585 B1**

(12) **EUROPEAN PATENT SPECIFICATION**

(45) Date of publication and mention
of the grant of the patent:
**18.03.2026  Bulletin 2026/12**

(21) Application number: **21720295.1**

(22) Date of filing: **23.04.2021**

(51) International Patent Classification (IPC):
**A61B 5/024** (2006.01)     **A61B 5/083** (2006.01)

(52) Cooperative Patent Classification (CPC):
**A61B 5/024; A61B 5/083;** A61B 5/681

(86) International application number:
**PCT/EP2021/060709**

(87) International publication number:
**WO 2021/219514 (04.11.2021 Gazette 2021/44)**

(54) **METHOD AND SYSTEM FOR PREDICTING A VO2MAX MEASUREMENT**

VERFAHREN UND SYSTEM ZUR VORHERSAGE EINER VO2MAX-MESSUNG

PROCÉDÉ ET SYSTÈME DE PRÉDICTION D'UNE MESURE DE VO2MAX

(84) Designated Contracting States:
**AL AT BE BG CH CY CZ DE DK EE ES FI FR GR HR HU IE IS IT LI LT LU LV MC MK MT NL NO PL PT RO RS SE SI SK SM TR**

(30) Priority: **27.04.2020  GB 202006149**

(43) Date of publication of application:
**08.03.2023  Bulletin 2023/10**

(73) Proprietor: **Myzone Limited**
**Isle of Man IM1 1EJ (GB)**

(72) Inventors:
• **WRIGHT, David Edward**
**Douglas, Isle of Man IM1 1EJ (GB)**
• **SANDERS, J.P.**
**Loughborough, Leicestershire LE11 3TU (GB)**
• **KINGSNORTH, A.P.**
**Loughborough, Leicestershire LE11 3TU (GB)**
• **ESILGER, D.W.**
**Loughborough, Leicestershire LE11 3TU (GB)**
• **THOMAS, Mr J.J.C.**
**Loughborough, Leicestershire LE11 3TU (GB)**

(74) Representative: **Mewburn Ellis LLP**
**Aurora Building**
**Counterslip**
**Bristol BS1 6BX (GB)**

(56) References cited:
• **DOMINIQUE HANSEN ET AL: "Validation of a single-stage fixed-rate step test for the prediction of maximal oxygen uptake in healthy adults", CLINICAL PHYSIOLOGY AND FUNCTIONAL IMAGING, vol. 36, no. 5, 5 June 2015 (2015-06-05), GB, pages 401 - 406, XP055764116, ISSN: 1475-0961, DOI: 10.1111/ cpf.12243**
• **NICOLE MAUE: "MZ-Fitness test", 5 April 2019 (2019-04-05), XP055765713, Retrieved from the Internet <URL:https://www.myzone.org/blog/ users/mz-fitness-test-2> [retrieved on 20210115]**
• **S WERNHART ET AL: "Correlation of heart rate recovery, aerobic physical activity and performance. A sub-analysis of the EURO-Ex trial", DEUTSCHE ZEITSCHRIFT FUER SPORTMEDIZIN, vol. 71, no. 1, 1 January 2020 (2020-01-01), DE, pages 19 - 24, XP055764206, ISSN: 0344-5925, DOI: 10.5960/dzsm.2019.402**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention relates to measuring cardiovascular fitness by predicting VO2max of a subject.

### Background

Cardiovascular disease (CVD) is the primary cause of mortality worldwide. Improvements in aerobic capacity have been associated with significant reductions in CVD risk in adults fitness (see Farrell SW, Kampert JB, Kohl HW, Barlow CE, Macera CA, Paffenbarger RS, Gibbons LW, Blair SN. Influences of cardiorespiratory fitness levels and other predictors on cardiovascular disease mortality in men. Med Sci Sports Exerc 1998;30(6):899-905. PMID:9624649). The most established method of quantifying cardiorespiratory fitness is measuring maximal oxygen uptake, otherwise known as $\dot{V}O2max$. VO2max is the measurement of the maximum amount of oxygen a person can utilize during intense exercise. However, maximal exercise testing may not be safe or practical for all individuals depending on their health and/or physical capacity. Furthermore, $\dot{V}O2max$ testing requires a number of specialist pieces of equipment and technically trained personnel. Due to the limitations of time, laboratory space and specialised equipment and the potential need for medical supervision, submaximal exercise tests have been proposed to be used to quickly analyse and predict $\dot{V}O2max$.

There are many different types of submaximal exercise tests, depending on age range and method of exercise (e.g. cycling or running) available and furthermore, they do not require the specialist equipment required by a maximal oxygen uptake test. However, given the ease with which submaximal testing can be performed, measures of fitness are not readily used by the general public. The developing arena of wearable technology may help with this issue.

When we begin to exercise, our sympathetic nervous system (i.e "fight-or-flight" mode) activates and our parasympathetic nervous system (i.e. "rest-and-digest" mode) slows. This will result in an increase in heart rate (HR), along with other physiological responses associated with preparing the body for- and sustaining exercise (e.g. increased blood flow to muscle and reduced blood flow to the digestive organs). Upon cessation of exercise, the opposite occurs, causing a decrease in our heart rate and a return of our body towards homeostasis. Our bodies ability to switch between these antagonistic systems can have significant consequences for our health. Several research groups have shown that HRR is regulated by cardiac autonomic activity, and in particular, the ability of our parasympathetic nervous system to reactivate post-exercise is an important indicator of our cardiovascular health and fitness. The quantification of heart rate decrement after exercise is known as heart rate recovery (HRR).

Dominique Hansen ET AL.: "Validation of a single-stage fixed rate step test for the prediction of maximal oxygen uptake in healthy adults" proposes an equation to estimate VO2max from a fixed-rate step test.

Nicole Maue: "MZ-Fitness test" (Retrieved from https:/www.myzone.org/blog/users/mz-fitness-test-2 https:/www.myzone.org/blog/page/3) proposes a Fitness test to offer a convenient and accurate measure of heart rate recovery.

S Wernhart ET AL: "Correlation of heart rate recovery, aerobic physical activity and performance. A sub-analysis of the EURO-Ex trial" proposes that HRR5 is a better predictor for maximal exercise capacity than HRR1 or HRR3.

The present invention has been devised in light of the above considerations.

### Summary of the Invention

The invention is set out in the appended claims.

By using a fitness test exercise with the subject's heart rate maintained in predetermined bands for predetermined durations of time, the inventors have found that VO2max can be calculated more reliably than tests in which subjects complete a predetermined activity, for example over a certain time or distance. The method may comprise displaying the predicted VO2max measurement to the subject. The measurement of VO2max may be absolute or relative VO2max.

The method may be a computer-implemented method which is performed by a device or system which may include one or more of: a smartphone, a tablet, a computer, a server and a heart rate sensor.

Collecting the HRR value may include receiving heart rate data from a heart rate monitor/sensor. The heart rate sensor may be an electrocardiogram (ECG) sensor, such as the Myzone® chest belt, or a PPG sensor.

The test may be performed using any type of exercise. It may be advantageous to perform the test by running or cycling as these activities are easily adjusted to ensure the correct heart rate is attained and maintained. The fitness test exercise may be undertaken on any cardiovascular apparatus which provides for the subject exerting themselves at varying intensities of their maximum heart rate.

The heart rate bands may be defined by a range of percentage of maximum heart rate. One of the phases may be a low intensity phase associated with a heart rate band that has an upper threshold of less than 70% of maximum heart rate. The upper threshold of the heart rate band of the low intensity phase may be between 40 and 65%, preferably between 55% and 65% and may be 60%. The low intensity phase may be the first phase to be performed in the fitness test exercise. One of the phases may be a high intensity phase associated with a heart rate band that has a lower threshold of greater than 70% of maximum heart rate. The high intensity phase may therefore have a higher heart rate range than the low intensity phase. The lower threshold of the high intensity phase may be between 70% and 85%, and may be 80%. An upper threshold of the high intensity phase may be between 80% and 95%, preferably between 80% and 90%, and may be 85%. The high intensity phase may be the last phase to be performed in the fitness test exercise.

The phases may be performed sequentially without rest. The heart rate ranges may increase in intensity over the test, so that the first phase has the lowest heart rate range and the final phase has the highest heart rate range. The first phase may have a range that is less than a first threshold percentage of the subject's maximum heart rate. Phases may have a range that is between the upper threshold of the previous phase and the lower threshold of the subsequent phase.

Each phase may take place for a predetermined amount of time. The test may have a total time of 10 minutes. Each phase may be performed for one or three minutes. Heart rate recovery may be measured for up to ten minutes, preferably for five minutes.

An example of a fitness test includes four phases. The first phase has a heart rate range of <60% or 50-59% max heart rate and is performed for one minute. The second phase has a heart rate range of 60-69% max heart rate and is performed for three minutes. The third phase has a heart rate range of 70-79% max heart rate and is performed for three minutes. The fourth phase has a heart rate range of 80-85% max heart rate and is performed for three minutes.

The method may comprise collecting one or more factors or values associated with the subject. Collecting the factors/values may include one or more of receiving a user input, retrieving a stored value, performing a measurement and receiving a measurement from a sensor.

The method may further comprise collecting a mass factor of the subject and the step of calculating the predicted VO2max measurement includes using the mass factor.

The mass factor may be the fat free mass of the subject. Fat free mass is a well-known measurement in elite sport and sports science, but may not be commonly measured by amateur fitness enthusiasts. So, collecting the fat free mass may comprise collecting a body fat percentage of the subject and a weight of the subject and calculating the fat free mass. Body fat percentage is a metric that is more easily measured and more commonly used by fitness enthusiasts. The fat free mass of the subject may alternatively be collected directly.

The fat free mass may not be commonly known among casual fitness enthusiasts, so an alternative method can utilise the subject's weight and sex to calculate a mass factor of the subject, instead of their fat free mass. This gives a less accurate, but more easily usable method for subjects who do not know their fat free mass or body fat percentage. In this alternative method, the method may comprise collecting a weight of the subject and the sex of the subject.

The method may further comprise collecting an ethnicity value, wherein the ethnicity value is a numerical value dependent on the ethnicity of the subject and the step of calculating the predicted VO2max measurement includes using the ethnicity value.

The first period may be at least 1 minute. The first period may be 2 minutes or 3 minutes.

The method comprises collecting a second heart rate recovery value measured over a second period starting at the end of the fitness test exercise, the second period being shorter than the first period and the step of calculating the predicted VO2max measurement includes using the second heart rate recovery value. The second period may be 1 minute, when the first period is longer than one minute.

The method may further comprise collecting one or more further heart rate recovery value(s) measured over further periods starting at the end of the fitness test exercise, and the step of calculating the predicted VO2max measurement may include using the further heart rate recovery value(s).

The method may further comprise collecting a power value measured during the fitness test exercise and the step of calculating the predicted VO2max measurement may include using the power value. The power value may be measured over an end portion of the fitness test exercise. The power value may be measured over the last 30 seconds of the fitness test exercise. The power value may be measured over a period of the fitness test exercise in which the subject exerts maximal effort. This period may be a phase of the fitness test in which the heart rate range is highest. The power value may be a wattage or speed measured by exercise equipment used in the fitness test exercise.

The step of calculating the predicted VO2max measurement may comprise: applying a respective weighting to each of the values, and adding the weighted values.

The step of calculating the predicted VO2max measurement may further comprise: adding a baseline value.

The weighting for heart rate recovery value may be 0.02 +/- 0.015 L/min / bpm, and/or a white ethnicity may be given a value of 1 and the weighting for ethnicity of the subject is -0.35 +/- 0.2 L/min. The weighting for fat free mass may be 0.055 +/- 0.025 L/min / kg. The weighting for the subject's weight may be 0.025 +/- 0.02 L/min / kg and/or a male may be given a value of sex of 1 and the weighting for the subject's sex is 0.822 +/- 0.3 L/min. The weighting for the second heart rate recovery value may be -0.02 +/- 0.01 L/min / bpm. The weighting for the power value may be 0.006 +/- 0.003 L/min / W.

Alternative units may be used, but the value should either be converted into the units given in the equation to be used or the weighting in the equation should be converted to match the units of the data input. Where numerical values are assigned to non-numerical parameters (e.g. sex, ethnicity), the values assigned may be changed with an appropriate change to the weighting and/or baseline value, so that the parameters affect the predicted VO2max equation in the same way.

In the step of calculating the predicted VO2max, heart rate recovery value may have a positive effect on predicted VO2max, such that a higher heart rate recovery value will cause a higher predicted VO2max. Fat free mass may have a positive effect on predicted VO2max, such that a higher fat free mass will cause a higher predicted VO2max. When the ethnicity is white, the predicted VO2 max may be increased. The second heart rate recovery value may have a negative effect on the predicted VO2max such that a higher second heart rate recovery value will cause a lower predicted VO2max. When the sex is male, the predicted VO2 max may be increased. Weight may have a positive effect on the predicted VO2max, such that a higher weight will cause a higher predicted VO2max.

The step of calculating the predicted VO2max may include performing one of the following equations:

- Predicted VO2max = 3.230 - (HRR1 x 0.002)
- Predicted VO2max = 2.123 + (HRR2 x 0.020)
- Predicted VO2 max = 2.222 - (HRR1 x 0.032) + (HRR2 x 0.040)
- Predicted VO2max = (fat free mass x 0.045) - (ethnicity x 0.236) + (HRR2 x 0.014) - (HRR1 x 0.016) + (Avg_Watts x 0.006) - 0.243
- Predicted VO2max = *(fat free mass x 0.066) - (Ethnicity x 0.357) + (HRR2 x 0.019) - (HRR1 x 0.017) - 0.729*
- Predicted VO2max = *(Weight x 0.025) - (Ethnicity x 0.456) + (HRR2 x 0.029) - (HRR1 x 0.022) + (Sex x 0.822) - 0.330*

Wherein HRR2 is heart rate recovery over two minutes after the end of the fitness test exercise and HRR1 is heart rate recovery over one minute after the end of the fitness test exercise. Avg_watts is the average wattage from an exercise bike used to complete the fitness test exercise over the final thirty seconds of the fitness test exercise.

The method may further comprise comparing the predicted VO2max with a database to determine a fitness age, wherein the database contains a value for VO2max for each integer age in years and the fitness age is determined to be the age which has a VO2max value that is closest to the predicted VO2max. This score may be more relatable to a casual fitness enthusiast. The database may be derived from a normative database. The value for VO2max may be an average of VO2max for subjects of the corresponding age. The value for VO2max may be average data for a percentile of VO2max for subjects of the corresponding age. The database may include multiple values for each age, such as the 30%, 50% and 75% percentiles. The method may further comprise displaying to a subject their predicted VO2max, a VO2max value from the database for their age and the subject's calculated fitness age. The database may be split into values for each sex and the fitness age may be determined to be the age which has a VO2max value that is closest to the predicted VO2max for the subject's sex.

The method may comprise, during each phase of the fitness test exercise, instructing the subject to adjust their efforts to maintain their heart rate in the heart rate band corresponding to the respective phase of the fitness test exercise. The method may further comprise, during each phase of the fitness test exercise, displaying the respective heart rate band. The method may further comprise displaying the subject's heart rate during the fitness test exercise. These features may assist the user in maintaining their heart rate in the respective heart rate bands as they perform the fitness test exercise.

Collecting the heart rate recovery value may comprise the steps of: detecting the end of the fitness test exercise completed by the subject, automatically measuring heart rate data over the first (and optionally second) period, and calculating the heart rate recovery value.

As discussed previously, a method according to the description above may be a computer-implemented method which is performed by a device or system which may include one or more of a computing device, a server and a heart rate monitor/sensor. The computing device may be a smartphone, a tablet, or a personal computer. The components may communicate with one another via wired and/or wireless communication channels.

Preferably, the step of calculating a predicted VO2max (and optionally collecting the one or more factors) is performed by a computing device. In alternative embodiments, a server in communication with the computing device may perform the calculation of the predicted VO2max, and optionally the collection of one or more of the factors. The heart rate monitor may be in communication with a computing device and the computing device may be in communication with a server. Preferably, the heart rate monitor communicates with the computing device via Bluetooth. Preferably, the computing device communicates with the server via the internet, for example via WiFi.

Preferably, the heart rate monitor measures heart rate of the subject during the fitness test exercise and during a recovery period after the fitness test exercise. The heart rate monitor may send the heart rate data to the computing device. The computing device may display the heart rate data in real time to aid the subject with adjusting their exertion to maintain the target heart rate during the fitness test exercise. The computing device may calculate the heart rate recovery value(s) based on the heart rate data received from the heart rate monitor. The computing device may collect the other factors and calculate the predicted VO2max and may display this to the user. The computing device may send the predicted VO2max to the server for storing in a database. The collected factors and heart rate data may also be stored in the database at the server.

Factors such as the mass factor, sex and/or ethnicity of the subject may be stored at a server. The computing device may also send updates to the stored factors to the server when available (for example when input by the user). Heart rate data may be measured using a heart rate monitor and the heart rate recovery value may be calculated at the heart rate monitor, the computing device or the server.

The computing device may be a smartphone. When the fitness test exercise is performed in a specialised fitness facility, such as a Myzone® enabled club, the computing device may be a computer with one or more display(s).

In another aspect, the invention provides a system for predicting a VO2max measurement for a subject, the system comprising a computing device and a heart rate monitor, the system configured to: collect a heart rate recovery value, measured over a first period starting at the end of a fitness test exercise completed by the subject, wherein the fitness test exercise comprises a plurality of phases, each phase having a predetermined duration and each phase being associated with a respective heart rate band, and during each phase, the subject adjusts their effort to maintain their heart rate in the respective heart rate band, and calculate a predicted VO2max measurement using the heart rate recovery value.

The system may be configured to perform any of the above discussed method features.

The system may further comprise a server. The computing device may be a smartphone and/or a personal computer. The components may communicate with one another via wired and/or wireless communication channels.

Preferably, the computing device is configured to calculate the predicted VO2max (and optionally collect one or more of the factors). In alternative embodiments, the server is in communication with the computing device and the server may be configured to calculate the predicted VO2max (and optionally collect one or more of the factors). The heart rate monitor may be in communication with the computing device and the computing device may be in communication with the server. Preferably, the heart rate monitor is configured to communicate with the computing device via Bluetooth. Preferably, the computing device is configured to communicate with the server via the internet, for example via WiFi.

Preferably, the heart rate monitor is configured to measure heart rate of the subject during the fitness test exercise and during a recovery period after the fitness test exercise. The heart rate monitor may be configured to begin measuring heart rate when triggered by the computing device or may automatically begin measuring heart rate data when a signal is provided by the subject, for example when ECG signals are detected by the heart rate monitor.

The heart rate monitor may be configured to send the heart rate data to the computing device in real time, and/or when connected to the computing device. The computing device may be configured to display the heart rate data in real time. This may aid the subject with adjusting their exertion to maintain the target heart rate during the fitness test exercise. The computing device may be configured to calculate the heart rate recovery value(s) based on the heart rate data received from the heart rate monitor. The computing device may be configured to collect the other factors and calculate the predicted VO2max and may display the predicted VO2max to the user. The computing device may be configured to send the predicted VO2max to the server for storing in a database. The collected factors and heart rate data may also be stored in the database at the server.

Factors such as the mass factor, sex and/or ethnicity of the subject may be stored at the server. The computing device may also be configured to send updates to the stored factors to the server when available (for example when input by the user). Heart rate data may be measured using a heart rate monitor and the heart rate recovery value may be calculated at the heart rate monitor, the computing device or the server.

The computing device may be a smartphone. When the fitness test exercise is performed in a specialised fitness facility, such as a Myzone® enabled club, the computing device may be a computer with one or more display(s).

In another aspect, the invention provides a computer program configured, when run on a computing device, to cause the computing device to perform the method as described above. The computer program may be an application for a smartphone, tablet or computer.

The invention includes the combination of the aspects and preferred features described except where such a combination is clearly impermissible or expressly avoided.

Summary of the Figures

Embodiments and experiments illustrating the principles of the invention will now be discussed with reference to the accompanying figures in which:

**Figure 1a** shows an example of a system configured to predict a subject's VO2max.
**Figure** 1b shows an example of a system configured to predict a subject's VO2max.

**Figure** 2 shows an example heart rate graph for a subject performing a fitness test exercise and recovery period.

Detailed Description of the Invention

Aspects and embodiments of the present invention will now be discussed with reference to the accompanying figures. Further aspects and embodiments will be apparent to those skilled in the art.

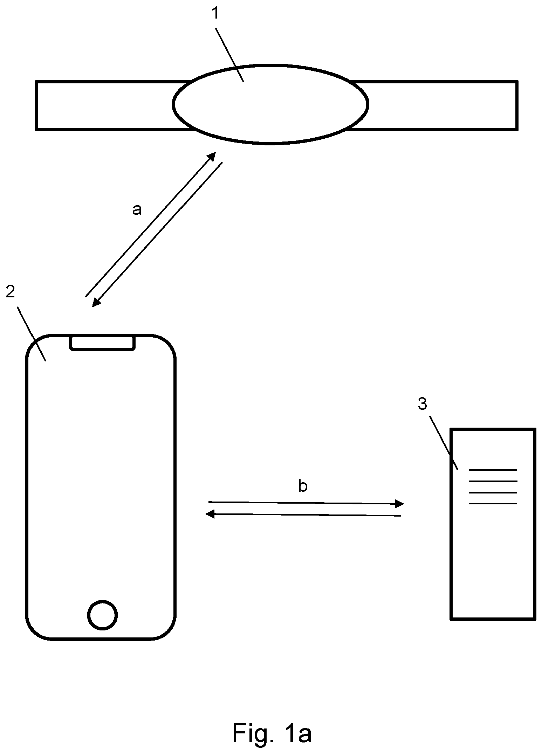

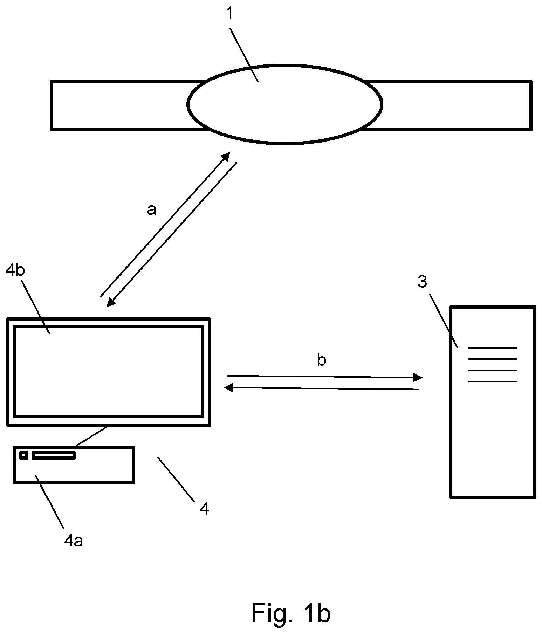

Two example systems which may perform the method of the present invention are shown in Figures 1a and 1b. Each system includes a heart rate monitor 1, a computing device and a server 3. The system of Figure 1a includes a smartphone 2 as the computing device and the system in Figure 1b shows a computer 4 with a tower 4a and a display 4b. The tower may be integrated with the display, for example in a smart-TV screen. Other types of computing devices may be used in place of those shown without departing from the present invention.

A heart rate monitor 1 is provided to be worn by the subject. The heart rate monitor 1 may be a Myzone® chest belt monitor or another type of heart rate monitor which may be chest-based or wrist-based. The heart rate monitor 1 may have a memory for storing heart rate data.

The heart rate monitor 1 has a communication module configured to communicate with the computing device as shown by the arrows a. This may be by a wired connection or by wireless communication such as WiFi or Bluetooth. The heart rate monitor is configured to send heart rate information, such as heart rate in real time, stored heart rate data, or calculated heart rate recovery data to the computing device. Where the heart rate monitor sends HRR data to the computing device, the heart rate monitor calculates this from heart rate data measured over the first period starting at the end of a fitness test exercise completed by the subject.

Calculating the heart rate recovery value from the heart rate data may include analysing the heart rate data to determine the end of the fitness test exercise, determining the heart rate at the end of the fitness test exercise, determining the end of the first period and determining the heart rate at the end of the first period and subtracting the heart rate at the end of the first period from the heart rate at the end of the fitness test exercise to determine the heart rate recovery value.

The computing device has a processor, a memory, a display and a user input which may be a keyboard, mouse and/or touch screen. The computing device is configured to communicate with the heart rate monitor via the arrows a by a wired connection or by wireless communication such as WiFi or Bluetooth. The computing device is also configured to communicate with server 3 via arrows b. This connection may be a wired or wireless communication channel and may include communication over the internet.

The computing device has software installed which enables the system to collect factors and calculate the predicted VO2max. The software may be an application for smartphone 2 or a computer program for tower 4a. The software may enable the computing device to carry out all of the method or may enable the computing device, together with the server to carry out the method.

The computing device is configured to receive data from the heart rate monitor 1. Where the heart rate data includes the heart rate recovery value, this amounts to collection of the heart rate recovery value. Where raw heart rate data is received from the monitor, the computing device may calculate the heart rate recovery value from the heart rate data. In this case, calculating the heart rate recovery value constitutes collecting the heart rate recovery value.

The software may be configured such that the user may input factors directly via the user input(s), or it may allow a user to input information that the computing device can use to determine a factor. For example, when the mass factor is fat free mass, the user may input body fat percentage and weight and the computing device may calculate the fat free mass using this information.

The computing device may store information about factors/values, or factors themselves or any other information about the subject, including the predicted VO2max values in the memory of the computing device or at the server 3 so that the user need not enter them every time the method is to be performed. In this case, collecting the factors may comprise retrieving the stored information from the memory or the server 3. The server may act as a store or back-up for information about the subject.

In some embodiments, the computing device may send factors or information from which factors can be determined to the server 3, and the server 3 will collect the factors and calculate the predicted VO2max. In this case, collecting the factors comprises the server receiving factors from the computing device, the server determining factors from information or the server retrieving stored factors.

The system in Figure 1a has the advantage that it can be used by a single user without specialist equipment as the necessary software (e.g. an application) can simply be installed on their smartphone to enable the method of the present invention to be performed. The system in Figure 1b may be used in a club environment where many subjects may carry out the fitness test exercise. The software required may be installed on the tower 4a.

The system may further comprise a piece of exercise equipment in communication with the computing device. In this case, the exercise equipment may send the computing device information about power attained during the exercise to enable the power factor to be determined. Alternatively, the exercise equipment may display this value and the user may enter the power factor manually into the computing device.

*Example*

An example of performing the method of the present invention will now be described.

A subject wears heart rate monitor 1 while performing a fitness test exercise in which their heart rate is increased from 50-59% of their maximum HR at the beginning of the test to 80-85% of their maximum heart rate over 10 minutes. The smart phone 2 may display the target heart rate to instruct the user to attain the target heart rate. They then rest for the following five minutes.

The heart rate monitor 1 measures the subject's HR throughout the fitness test and during the recovery period. The heart rate monitor stores the heart rate data in its memory.

When a connection is present between the smartphone 2 and the heart rate monitor, the heart rate monitor sends the heart rate data to the smartphone. This may be in real time as the subject carries out the fitness test exercise, or after the exercise has been completed. The HR of the subject may be displayed by the smartphone in real time, optionally in addition to the target heart rate range, to aid the subject in attaining the target heart rate during the test exercise.

An app running on the smartphone 2 retrieves data stored about the subject from the smartphone memory or from the server 3. The app asks the subject to input their fat free mass in kg or body fat percentage and weight in kg and their ethnicity or retrieves previously stored values for these factors. Alternative units may be used, but the value should either be converted into the units given in the equation to be used or the weighting in the equation should be converted to match the units of the data input.

If body fat percentage and weight are entered, the fat free mass is calculated using the equation:

fat free mass = weight x (1 - body fat percentage).

If the subject does not know their body fat percentage or fat free mass, they are asked to enter their weight and sex.

The app calculates the first HRR (HRR2) from the heart rate data by analysing the heart rate data to determine the end of the fitness test exercise, determining the heart rate at the end of the fitness test exercise, determining the heart rate at the end of the second minute of recovery and subtracting the heart rate at the end of the second minute of recovery from the heart rate at the end of the fitness test exercise to determine the first heart rate recovery value (HRR2).

The app calculates the second HRR (HRR1) from the heart rate data by analysing the heart rate data to determine the end of the fitness test exercise, determining the heart rate at the end of the fitness test exercise, determining the heart rate at the end of the first minute of recovery and subtracting the heart rate at the end of the first minute of recovery from the heart rate at the end of the fitness test exercise to determine the second heart rate recovery value (HRR1).

If the fat free mass was entered, retrieved or calculated, then formula 1 below is used to determine the predicted VO2max. If the fat free mass was not entered, retrieved or calculated, then formula 2 below is used to determine the predicted VO2max.

$$VO2max = (fat\ free\ mass\ x\ 0.066) - (Ethnicity\ x\ 0.357) + (HRR2\ x\ 0.019) - (HRR1\ x\ 0.017) - 0.729$$

VO2max = (*Weight* x 0.025) - (*Ethnicity* x 0.456) + (*HRR2* x 0.029) - (*HRR1* x 0.022) + (*Sex* x 0.822) - 0.330

The predicted VO2max is displayed to the user on the computing device and sent to the server for storage.

*Experiments*

The following examples show the accuracy of three methods according to the present invention. In the examples, a method according to the present invention is compared with a VO2max test to show that results achieved with a method according to the present invention are reliable predictions of the VO2max of a subject.

In each example, measures of height, weight, body fat percentage, fat free mass and fat mass were obtained. Measures of height were measured to the nearest 0.1 cm and measures of weight were obtained to the nearest 0.05kg.

To measure VO2max, a computerised metabolic system (Cortex Metalyser 3B-R2, Cortex Biophysik GMBH, Leipzig, Germany) measured VO2 breath-by-breath with a flowmeter connected to a Hans-Rudolf mask. Before each test barometric pressure and ambient air was measured, and the gas analyser and inspiratory flowmeter were calibrated.

HR during the tests was measured using the MZ-3 Chest Heart Rate Monitor (Myzone Ltd, Isle of Man, UK), with a Myzone Receiver (MZ-R) used to log the HR data during the MZFitness test in one-second epochs.

**Example 1**

**Cycling MZ-Fitness Test**

All tests were performed in a laboratory environment in a thermo-neutral environment. For the cycling MZ-Fitness Test, a Wattbike Trainer (Wattbike, Nottingham, UK) was used. Participants were familiarised with the Wattbike, both in terms of the seat and handlebar adjustment and how to alter the resistance using airflow and magnetic resistance. The MZ-Fitness Test described below is a sub-maximal test which required the participants to exercise through different HR zones for 10 minutes (see Table below), by manipulating the Wattbike's resistance and/or their cadence to achieve a HR within the correct HR Zone for that period of the test. The HR zones are percentages of age predicted heart rate max (HRmax) using the HUNT study equation for predicting HRmax which is 211 - 0.64 x age.

The HR zones are colour coded as shown in the table below.

| Zone | Heart Rate | Time (mins) |
|---|---|---|
| Grey | 50-59% | 1 |
| Blue | 60-69% | 3 |
| Green | 70-79% | 3 |
| Purple | 80-85% | 3 |
| Recovery | HRR | 5 |
| | **Total time** | **15** |

A verbal explanation of the test was provided to the participants which was followed by a three-minute warm-up period. During this time, the participant was instructed to try and get themselves into the first HR Zone (50-59% of age predicted HRMax). This ensured patient safety as well as enabling familiarisation with the Myzone app, test screen on the app, and the practical use of the Wattbike. A washout period followed to enable participants to stretch, after which the MZ-Fitness Test began.

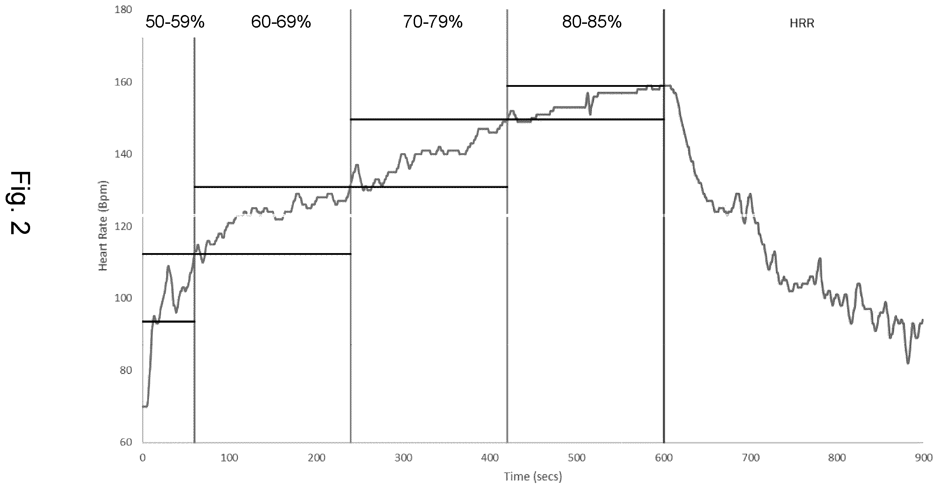

At the end of the test, the participant's HR was recorded for five minutes. Participants were asked to stop cycling entirely and not to "spin their legs" as may be the traditional instruction following a cycle MZ Fitness test validation ergometer fitness test. The first minute of the recovery period was conducted with the participant still seated on the Wattbike. After this initial minute, participants were asked to move to a chair and remain seated for a further four minutes. Figure 2 displays exemplar data of a participants HR during the MZFitness test and subsequent recovery period.

HRR was determined as the absolute difference in final heart rate post submaximal test and after 1, 2, 3, 4, and 5 minutes. The HRR after n minutes will be referred to as HRRn to avoid confusion, for example, the HRR after one minutes will be referred to as HRR1 and so on.

**Cycling V̇O2max Test**

A maximal cycle ergometer ramp test on a Lode Sport Excalibur Cycle Ergometer (Lode BV Medical Technology, Groningen, Netherlands) was used to determine V̇O2max. After a short rest period after the MZ-Fitness Test, the cycling max test began. The test began with a two-minute period of resting whilst sitting on the ergometer, followed by a three-minute warm-up which led directly into the ramp test. The ramp test was determined using a previously validated prediction equation to estimate VO2max without the need for previous exercise data (see equation 1)

$$Predicted\ VO_{2max}$$
$$= 34.142 - 0.005(Age^2) + 11.403(Gender) + 1.463(PAS) + 9.170(Height)$$
$$- 0.254(Body\ Mass)$$

*Where*

$$Gender\ (male = 1, female = 0); PAS, physical\ activity\ status\ (0 - 7)$$

Equation 1 - Matthews et al non exercising VO2max prediction equation - see Matthews CE, Heil DP, Freedson PS, Pastides H. Classification of cardiorespiratory fitness without exercise testing. Med Sci Sports Exerc 1999;31(3):486-493. PMID:10188755.

Using the result of the above equation the cycling test ramp was determined using the ACSM cycling metabolic equations to calculate the cycle ergometer peak work rate (See Equations 2-4).

$$Power_{Final} = \left( \frac{((Predicted\ VO_{2 \cdot max} \times Body\ Mass) - 7)}{1.8} \right) / 6.12$$

Equation 2 - ACSM Cycling Metabolic Equations to calculation predicted final/peak power during a cycling ergometer test (see Cunha FA, Midgley A, Montenegro R, Vasconcellos F, Farinatti P. Utility of a Non-Exercise VO2max Prediction Model for Designing Ramp Test Protocols. Int J Sports Med 2015;36(10):796-802. [doi: 10.1055/s-0034-1395590]).

Depending on the participant's capabilities in the MZ-Fitness Test, participants began their ramp test at either 30 or 70 Watts (W). If a participant's final power during the MZ-Fitness was less than or equal to 90W, participants began their maximal test at 30W. This was to ensure that participants could complete the test and would not finish the test prematurely. This decision was made at the discretion of the investigators.

$$Power_{Initial} = 30\ or\ 70\ W$$

Equation 3 - Modified ACSM Initial Power

Therefore, the ramp during the test was determined as follows:

$$Ramp\ Ratio = \frac{Power_{Final} - Power_{Initial}}{10min}$$

Equation 4 - ACSM equation to determine the ramp protocol for the cycling ergometer max test (see Cunha FA, Midgley A, Montenegro R, Vasconcellos F, Farinatti P. Utility of a Non-Exercise VO2max Prediction Model for Designing Ramp Test Protocols. Int J Sports Med 2015;36(10):796-802. [doi: 10.1055/s-0034-1395590]). Note: the equation was based on a predicted 10 minute test however, the test was open ended and finished based on the criteria described below.

During the ramp portion of the V̇O2max test, participants were instructed to cycle at a consistent cadence between 70-85 rpm for as long as they could. During each minute of the ramp, participants were asked to provide their Rating of Perceived Exertion (RPE) using the Borg10 Scale (see Borg G. An introduction to Borg's RPE-scale. Ithaca, NY Mouv Publ. 1985. and Borg E, Kaijser L. A comparison between three rating scales for perceived exertion and two different work tests. Scand J Med Sci Sport 2006;16(1):57-69. [doi: 10.1111/j.1600-0838.2005.00448.x]).

The test was terminated when one of the three conditions were met:

- The cadence dropped by 10 rpm for over 10 seconds,
- Participants volitionally ceased exercise or
- The researcher deemed termination was necessary due to health and safety reasons

At the end of the test, the same recovery protocol was followed as performed during the MZ-Fitness Test.

**Statistical Analysis**

Breath-by-breath V̇O2max data collected during each ramp test were time-averaged over 30 seconds. A participant was determined to meet V̇O2max if the following criteria were satisfied:

- Oxygen Uptake plateaued with an increase in workload
- HR plateau with an increase in heart rate
- Max HR was within $\pm 15$ BPM of age-predicted Max HR
- Respiratory Exchange Ratio of $\geq 1.1$
- Borg10 $\geq 7$

There is no consensus on the number of criteria that should be met in order to call a test maximal, however, the inventors used the plateau in either heart rate or oxygen uptake as a criterion to determine whether max had been reached.

**Prediction equation used:**

$$VO2max = (fat\ free\ mass \times 0.045) - (Ethnicity \times 0.236) + (HRR2 \times 0.014) - (HRR1 \times 0.016) \\ + (Avg\_watts \times 0.006) - 0.243$$

Where: fat free mass measured in kg, ethnicity (white=1, non-white=0), HRR2 is the heart rate recovery after 2 minutes, HRR1 is the heart rate recovery after 1 minute and Avg_watts is the average watts in the final 30 seconds of the purple zone.

Equation 5. VO2max prediction equation that includes a power-based metric.

This equation explained a total of 93.6% of the variance in $\dot{V}$O2max with a Standard error of estimate (SEE) of 0.22 L.min. This equation uses a participant's fat free mass, their ethnicity, their HRR after 1 and 2 minutes of the MZ-Fitness Test and the average watts in the final 30 seconds.

### Example 2

In this example, the same fitness tests were performed as in Example 1. However, in this example, the power-based metric, or power value is omitted so as to make a prediction possible using a fitness test in conditions where it is not possible or it is difficult to measure or collect the power-based metric.

**Prediction equation used:**

$$VO2max = (fat\ free\ mass \times 0.066) - (Ethnicity \times 0.357) \\ + (HRR2 \times 0.019) - (HRR1 \times 0.017) - 0.729$$

Where: fat free mass measured in kg, ethnicity (white=1, non-white=0), HRR2 is the heart rate recovery after 2 minutes, HRR1 is the heart rate recovery after 1 minute.

Equation 6. VO2max prediction equation without a power based metric.

This equation explains 88.8% of the variance in $\dot{V}$O2max with a standard error of the estimate (SEE) of 0.29 L.min. This means that 95% of the time, an individual's predicted VO2max is accurate to $\pm$ 0.29 L.min.

In comparison with the equation used in example 1, the removal of average watts in the final 30 seconds of the purple zone resulted in a decrease in explained variance of 4.8% from Equation 5. However, the variables present in Equation 6 are much more easily provided by the subject and therefore it is believed the decrease in explained variance is justified.

### Example 3

In this example, the same fitness tests were performed as in Example 1. However, in this example, the power-based metric, or power value is omitted so as to make a prediction possible using a fitness test in conditions where it is not possible to measure the power-based metric. Further, the fat free mass is not used and instead, weight and sex are included. The reason for this is that it can be difficult for individuals to obtain a measurement of their fat free mass. Therefore, although using weight may provide a less accurate prediction, it allows a prediction to be given even if an individual does not yet have a measurement of their fat free mass.

$$VO2max = (Weight \times 0.025) - (Ethnicity \times 0.456) + (HRR2 \times 0.029) - (HRR1 \times 0.022) \\ + (Sex \times 0.822) - 0.330$$

Where: weight is measured in kg, ethnicity (white=1, non-white=0), HRR2 is the heart rate recovery after 2 minutes, HRR1 is the heart rate recovery after 1 minute and self-reported sex (male=1, female =0). Equation 7. VO2max prediction equation if a subject does not know fat free mass.

In this equation, fat free mass is replaced by weight and sex (sex is coded as male=1, and female=0 in the equation) has also been reintroduced to the equation. This equation explained 82.3% of the variance in VO2max. This equation has a SEE of 0.36 L.min meaning 95% of the time the predicted VO2max will be within $\pm$ 0.36 L.min of the measured $\dot{V}$O2max. Sex has been introduced into the equation above. This can be explained as fat free mass explains the same variance as sex explains. However, weight does not explain this variance and therefore sex is reintroduced as a significant predictor into the equation.

### Further research

### Treadmill MZ-Fitness Test

The treadmill test was performed on an H/P/Cosmos Quasar Treadmill (H/P/Cosmos Sports and Medical GMBH, Nussdoorf, Germany). The test was performed in exactly the same way as the test during the cycling test, except during the treadmill test participants were asked to adjust their running speed and incline as they saw fit to make sure that they were in the right HR Zones. Furthermore, at the end of the test, participants remained standing for the first minute of the recovery period, before moving to a chair for a further 4 minutes.

### Treadmill $\dot{V}O2max$ Test

A maximal incline ramped treadmill test using the H/P/Cosmos Quasar Treadmill, was used to determine $\dot{V}O2max$. This protocol was adapted from Sport and Exercise Physiology Testing Guidelines. The test used the final running speed from the MZ-Fitness test, and subtracted 2km/h (or 1 km/h if subtracting 2km/h meant the participant would be walking for the max test) to determine the starting speed for the max test. Participants also began the max test at 1% incline, the incline which most accurately represents outdoor running. Following the resting and warm-up phase, the ramp protocol began, which involved increasing the incline by 1% gradually every minute. The max test was terminated volitionally by the participant at exhaustion.

The results given by a sub group of the participants in examples 1 to 3 and analysis assessing the agreement of the outcome from the MZ-Fitness test across exercising modalities showed moderate to strong significant correlations between HRR1 and HRR2 across both the treadmill and the cycle modes of testing ($r \leq 0.664$, $p \leq 0.05$).

The VO2max prediction can be calculated in the same manner as for the examples above.

### Comparison of VO2max with normative data

Once an individual is provided a prediction of their $\dot{V}O2max$, it is helpful to provide them additional information about how this value compares to other individuals. To do this, normative data sets can be used. Normative data sets can be used to provide an individual a categorisation, and percentage value, based on their age and sex. For example, if an individual aged 25 had a predicted VO2max of 48 ml.kg.min, they could be informed that their fitness was "Good" and in the top 30% of individuals for their age and sex.

There are a wide range of normative data sets published in previous literature. One example is the American College of Sports Medicine Fitness Registry and the Importance of Exercise National Database (ACSM FRIEND).

### Fitness Age

A Fitness Age score allows an individual to know whether their fitness is higher, or lower, than would be expected of an individual their age. To calculate this score, the ACSM FRIEND normative data set was used. Linear interpolation was performed between data points within this normative data set. This process involves predicting the data between two, or more, known data points by constructing a straight line between them. This allows a $\dot{V}O2max$ value to be assigned for each age point.

The database contains information for each 5% percentile of fitness for each age. So, the subject can choose to be compared with a certain percentile in the data. For example, someone with good fitness can be compared with data for those with good fitness. In this example, poor, fair and good categories were used. Good was assigned to the 75% percentile in the normative data, fair to the 50% data, poor to the 30% data.

The linear regression process was repeated for the poor, fair and good categories. This allows an individual to receive a Fitness Age score in comparison to a person who has poor, fair or good fitness. This allows a more meaningful comparison to be made as if an individual has a good fitness level comparing them to data relating to people with poor fitness may not be motivational. This allows the provision of a score that tells the subject whether they are more, or less, fit than someone with poor, fair or good fitness for their age. It can also provide an individual with what would be considered a good, fair or poor $\dot{V}O2max$ score for their age and sex.

For example, if a 25 year old male subject is compared with people with good fitness, and the average VO2max in the 75% percentile of the database for 25 year old males is 55 and the subject has a predicted VO2max of 55, then the fitness age score will be 25. If the same subject is compared with people with poor fitness, and the average VO2max in the 30% percentile of the database for 25 year old males is 42 and the subject has a predicted VO2max of 55, then the fitness age score will be younger than 25.

In another example, if a 55 year old female subject is compared with people with good fitness, and the average VO2max in the 75% percentile of the database for 55 year old females is 25 and the subject has a predicted VO2max of 25, then the fitness age score will be older than 55, for example, 60. If the same subject is compared with people with poor fitness, and the average VO2max in the 30% percentile of the database for 55 year old females is 21 and the subject has a predicted VO2max of 25, then the fitness age score may be younger than 55, for example, 42.

The systems and methods of the above embodiments may be implemented in a computer system (in particular in computer hardware or in computer software) in addition to the structural components and user interactions described.

The term "computer system" includes the hardware, software and data storage devices for embodying a system or carrying out a method according to the above described embodiments. For example, a computer system may comprise a central processing unit (CPU), input means, output means and data storage. Preferably the computer system has a monitor to provide a visual output display (for example in the design of the business process). The data storage may comprise RAM, disk drives or other computer readable media. The computer system may include a plurality of computing devices connected by a network and able to communicate with each other over that network.

The methods of the above embodiments may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

The methods of the above embodiments may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. The scope of the invention is defined by the appended claims.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example +/- 10%.

**Claims**

**1.** A method of predicting a VO2max measurement for a subject, the method comprising:

collecting a heart rate recovery value, measured over a first period starting at the end of a fitness test exercise completed by the subject, wherein the fitness test exercise comprises a plurality of phases, each phase having a predetermined duration and each phase being associated with a respective heart rate band, and during each phase, the subject adjusts their effort to maintain their heart rate in the respective heart rate band,
collecting a second heart rate recovery value measured over a second period starting at the end of the fitness test exercise, the second period being shorter than the first period, and
calculating a predicted VO2max measurement using the heart rate recovery value and the second heart rate recovery value.

2. A method according to claim 1, wherein the heart rate bands are defined by a range of percentage of maximum heart rate, and wherein the fitness test exercise comprises a low intensity phase and a high intensity phase, the low intensity phase having a lower heart rate range than the high intensity phase, and wherein the low intensity phase is the first phase to be performed in the fitness test exercise, and the high intensity phase is the last phase to be performed in the fitness test exercise.

3. A method according to claim 1 or claim 2, wherein the first period is at least 1 minute, and optionally wherein the first period is 3 minutes.

4. A method according to any preceding claim, wherein the second period is 1 minute.

5. A method according to any preceding claim, wherein the method further comprises:

collecting a mass factor of the subject, and the step of calculating the predicted VO2max measurement includes using the mass factor; and/or
collecting an ethnicity value, wherein the ethnicity value is a numerical value dependent on the ethnicity of the subject and the step of calculating the predicted VO2max measurement includes using the ethnicity value.

6. A method according to claim 5, wherein the step of calculating the predicted VO2max measurement comprises:

applying a respective weighting to each of the collected values, and
adding the weighted values.

7. A method according to claim 6, wherein the step of calculating the predicted VO2max measurement further comprises: adding a baseline value.

8. A method according to any preceding claim, wherein heart rate recovery value has a positive effect on predicted VO2max.

9. A method according to claim 4 or any of claims 5 to 8 when dependent on claim 4, wherein the second heart rate recovery value has a negative effect on the predicted VO2max.

10. A method according to any preceding claim, wherein the method further comprises, during each phase of the fitness test exercise:

instructing the subject to adjust their efforts to maintain their heart rate in the heart rate band corresponding to the respective phase of the fitness test exercise; and/or
displaying the respective heart rate band.

11. A method according to any preceding claim, wherein the method further comprises, displaying the subject's heart rate during the fitness test exercise.

12. A method according to any preceding claim, wherein collecting the heart rate recovery value comprises the steps of:

detecting the end of the fitness test exercise completed by the subject,
automatically measuring heart rate data over the first period, and
calculating the heart rate recovery value.

**Patentansprüche**

1. Verfahren zur Vorhersage einer VO2max-Messung für ein Individuum, wobei das Verfahren Folgendes umfasst:

Ermitteln eines Herzfrequenzerholungswerts, der über einen ersten Zeitraum gemessen wird, der am Ende einer vom Individuum ausgeführten Fitnesstest-Übung beginnt, wobei die Fitnesstest-Übung eine Vielzahl von Phasen umfasst, wobei jede Phase eine vorbestimmte Dauer aufweist und jede Phase einem entsprechenden Herzfrequenzband zugeordnet ist und wobei das Individuum während jeder Phase seine Anstrengung anpasst, um seine Herzfrequenz im entsprechenden Herzfrequenzband zu halten,

Ermitteln eines zweiten Herzfrequenzerholungswerts, der über einen zweiten Zeitraum gemessen wird, der am Ende der Fitnesstest-Übung beginnt, wobei der zweite Zeitraum kürzer ist als der erste Zeitraum, und

Berechnen einer vorhergesagten VO2max-Messung unter Verwendung des Herzfrequenzerholungswerts und des zweiten Herzfrequenzerholungswerts.

2. Verfahren nach Anspruch 1, wobei die Herzfrequenzbänder durch einen Prozentbereich der maximalen Herzfrequenz definiert sind und wobei die Fitnesstest-Übung eine Phase niedriger Intensität und eine Phase hoher Intensität umfasst, wobei die Phase niedriger Intensität einen niedrigeren Herzfrequenzbereich aufweist als die Phase hoher Intensität und wobei die Phase niedriger Intensität die erste Phase ist, die bei der Fitnesstest-Übung ausgeführt werden soll, und die Phase hoher Intensität die letzte Phase ist, die bei der Fitnesstest-Übung ausgeführt werden soll.

3. Verfahren nach Anspruch 1 oder Anspruch 2, wobei der erste Zeitraum zumindest 1 Minute dauert und wobei der erste Zeitraum gegebenenfalls 3 Minuten dauert.

4. Verfahren nach einem der vorangegangenen Ansprüche, wobei der zweite Zeitraum 1 Minute dauert.

5. Verfahren nach einem der vorangegangenen Ansprüche, wobei das Verfahren weiters Folgendes umfasst:

Ermitteln eines Gewichtsfaktors des Individuums, und wobei der Schritt des Berechnens der vorhergesagten VO2max-Messung die Verwendung des Gewichtsfaktors umfasst; und/oder
Ermitteln eines Ethnienzugehörigkeitswerts, wobei der Ethnienzugehörigkeitswert ein Zahlenwert ist, der von der Ethnienzugehörigkeit des Individuums abhängt, und der Schritt des Berechnens der vorhergesagten VO2max-Messung die Verwendung des Ethnienzugehörigkeitswerts umfasst.

6. Verfahren nach Anspruch 5, wobei der Schritt des Berechnens der vorhergesagten VO2max-Messung Folgendes umfasst:

Anwenden einer entsprechenden Gewichtung auf jeden der ermittelten Werte und
Addieren der gewichteten Werte.

7. Verfahren nach Anspruch 6, wobei der Schritt des Berechnens der vorhergesagten VO2max-Messung weiters Folgendes umfasst: Addieren eines Basislinienwerts.

8. Verfahren nach einem der vorangegangenen Ansprüche, wobei der Herzfrequenzerholungswert einen positiven Effekt auf den vorhergesagten VO2max hat.

9. Verfahren nach Anspruch 4 oder nach einem der Ansprüche 5 bis 8 in Abhängigkeit von Anspruch 4, wobei der zweite Herzfrequenzerholungswert einen negativen Effekt auf den vorhergesagten VO2max hat.

10. Verfahren nach einem der vorangegangenen Ansprüche, wobei das Verfahren weiters Folgendes während jeder Phase der Fitnesstest-Übung umfasst:

Anweisen des Individuums, seine Anstrengungen so anzupassen, dass es seine Herzfrequenz im Herzfrequenzband hält, das der entsprechenden Phase der Fitnesstest-Übung entspricht; und/oder
Anzeigen des entsprechenden Herzfrequenzbands.

11. Verfahren nach einem der vorangegangenen Ansprüche, wobei das Verfahren weiters das Anzeigen der Herzfrequenz des Individuums während der Fitnesstest-Übung umfasst.

12. Verfahren nach einem der vorangegangenen Ansprüche, wobei das Ermitteln des Herzfrequenzerholungswerts folgende Schritte umfasst:

Bestimmen des Endes der Fitnesstest-Übung, die vom Individuum ausgeführt wurde,
automatisches Messen der Herzfrequenzdaten während des ersten Zeitraums und
Berechnen des Herzfrequenzerholungswerts.

**Revendications**

1. Procédé de prédiction d'une mesure VO2max d'un sujet, le procédé comprenant les étapes consistant à :

   collecter une valeur de récupération de fréquence cardiaque, mesurée sur une première période commençant à la fin d'un exercice de test de condition physique terminé par le sujet, dans lequel l'exercice de test de condition physique comprend une pluralité de phases, chaque phase présentant une durée prédéterminée et chaque phase étant associée à une bande de fréquence cardiaque respective, et pendant chaque phase, le sujet ajuste son effort pour maintenir sa fréquence cardiaque dans la bande de fréquence cardiaque respective,
   collecter une seconde valeur de récupération de fréquence cardiaque mesurée sur une seconde période commençant à la fin de l'exercice de test de condition physique, la seconde période étant plus courte que la première période, et
   calculer une mesure de VO2max prédite en utilisant la valeur de récupération de fréquence cardiaque et la seconde valeur de récupération de fréquence cardiaque.

2. Procédé selon la revendication 1, dans lequel les bandes de fréquence cardiaque sont définies par une plage de pourcentage de fréquence cardiaque maximale, et dans lequel l'exercice de test de condition physique comprend une phase de faible intensité et une phase de forte intensité, la phase de faible intensité présentant une plage de fréquence cardiaque inférieure à la phase de forte intensité, et dans lequel la phase de faible intensité est la première phase à effectuer dans l'exercice de test de condition physique, et la phase de forte intensité est la dernière phase à effectuer dans l'exercice de test de condition physique.

3. Procédé selon la revendication 1 ou la revendication 2, dans lequel la première période est d'au moins 1 minute, et facultativement dans lequel la première période est de 3 minutes.

4. Procédé selon l'une quelconque des revendications précédentes, dans lequel la seconde période est de 1 minute.

5. Procédé selon l'une quelconque des revendications précédentes, **caractérisé en ce que** le procédé comprend en outre les étapes consistant à :

   collecter un facteur de masse du sujet, et l'étape de calcul de la mesure de VO2max prédite inclut l'utilisation du facteur de masse ; et/ou
   collecter une valeur d'ethnicité, dans lequel la valeur d'ethnicité est une valeur numérique dépendant de l'ethnicité du sujet, et l'étape de calcul de la mesure de VO2max prédite inclut l'utilisation de la valeur d'ethnicité.

6. Procédé selon la revendication 5, dans lequel l'étape de calcul de la mesure V02max prédite comprend les étapes consistant à :

   appliquer une pondération respective à chacune des valeurs collectées, et
   ajouter les valeurs pondérées.

7. Procédé selon la revendication 6, dans lequel l'étape de calcul de la mesure de VO2max prédite comprend en outre l'étape consistant à : ajouter une valeur de ligne de base.

8. Procédé selon l'une quelconque des revendications précédentes, dans lequel la valeur de récupération de la fréquence cardiaque présente un effet positif sur la VO2max prédite.

9. Procédé selon la revendication 4 ou l'une quelconque des revendications 5 à 8 lorsqu'elles dépendent de la revendication 4, dans lequel la seconde valeur de récupération de fréquence cardiaque présente un effet négatif sur la VO2max prédite.

10. Procédé selon l'une quelconque des revendications précédentes, dans lequel le procédé comprend en outre, pendant chaque phase de l'exercice de test de condition physique, les étapes consistant à :

demander au sujet d'ajuster ses efforts pour maintenir sa fréquence cardiaque dans la bande de fréquence cardiaque correspondant à la phase respective de l'exercice de test de condition physique ; et/ou afficher la bande de fréquence cardiaque respective.

11. Procédé selon l'une quelconque des revendications précédentes, dans lequel le procédé comprend en outre l'affichage de la fréquence cardiaque du sujet pendant l'exercice de test de condition physique.

12. Procédé selon l'une quelconque des revendications précédentes, dans lequel la collecte de la valeur de récupération de fréquence cardiaque comprend les étapes consistant à :

détecter la fin de l'exercice de test de condition physique effectué par le sujet,
mesurer automatiquement les données de fréquence cardiaque au cours de la première période, et
calculer la valeur de récupération de fréquence cardiaque.

Fig. 1a

Fig. 1b

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **FARRELL SW ; KAMPERT JB ; KOHL HW ; BARLOW CE ; MACERA CA ; PAFFENBARGER RS ; GIBBONS LW ; BLAIR SN.** Influences of cardiorespiratory fitness levels and other predictors on cardiovascular disease mortality in men.. *Med Sci Sports Exerc*, 1998, vol. 30 (6), 899-905
- **NICOLE MAUE**. *MZ-Fitness test*, https:/www.my-zone.org/blog/users/mz-fitness-test-2 https:/www.myzone.org/blog/page/3
- **MATTHEWS CE ; HEIL DP ; FREEDSON PS ; PASTIDES H.** Classification of cardiorespiratory fitness without exercise testing.. *Med Sci Sports Exerc*, 1999, vol. 31 (3), 486-493
- **CUNHA FA ; MIDGLEY A ; MONTENEGRO R ; VASCONCELLOS F ; FARINATTI P.** Utility of a Non-Exercise VO2max Prediction Model for Designing Ramp Test Protocols.. *Int J Sports Med*, 2015, vol. 36 (10), 796-802
- **BORG G.** An introduction to Borg's RPE-scale.. NY Mouv Publ., 1985
- **BORG E ; KAIJSER L.** A comparison between three rating scales for perceived exertion and two different work tests.. *Scand J Med Sci Sport*, 2006, vol. 16 (1), 57-69